# EUROPÄISCHE PATENTSCHRIFT

(11) **EP 1 014 867 B1**
(45) Veröffentlichungstag und Bekanntmachung des Hinweises auf die Patenterteilung: **15.12.2004**
(21) Anmeldenummer: 98941209.3
(22) Anmeldetag: 14.09.1998
(51) Int. Cl.: A61B 17/12

(54) **VORRICHTUNG FÜR DIE IMPLANTATION FADENFÖRMIGER MATERIALIEN**
DEVICE FOR IMPLANTING FILAMENTOUS MATERIALS
DISPOSITIF POUR L'IMPLANTATION DE MATERIAUX FILIFORMES

(30) Priorität: 16.09.1997 CH 218897
(43) Veröffentlichungstag der Anmeldung: 05.07.2000
(73) Patentinhaber: Lüscher, Patrick, 8330 Pfäffikon (CH)
(72) Erfinder: Lüscher, Patrick, 8330 Pfäffikon (CH)
(74) Vertreter: Menges, Rolf, Dipl.-Ing.
(86) Internationale Anmeldenummer: PCT/CH1998/000393
(87) Internationale Veröffentlichungsnummer: WO 1999/013781

(56) Entgegenhaltungen:
- EP-A- 0 734 697
- EP-A- 0 769 307
- WO-A-94/26175
- WO-A-97/19643
- US-A- 5 167 624
- US-A- 5 499 995

## Beschreibung

Die Erfindung betrifft eine Vorrichtung zum Applizieren eines fadenförmigen Implantatmaterials mit einem druckerzeugenden, ein Fluid enthaltenden Mittel, das mit einem Gehäuse in Verbindung steht, welches in einen Kanal mit einer distalen Öffnung mündet, durch die das sich im Gehäuse befindende und sich durch den Kanal erstreckende fadenförmige Material mittels des druckerzeugenden Mittels und des Fluids aus der Vorrichtung transportiert wird. Der zu applizierende Faden ist in das distale Ende des Kanals eingeführt. Der durch das druckerzeugende Mittel auf das Fluid ausgeübte Druck ermöglicht den Transport des im Gehäuse befindlichen, zu implantierenden Fadens durch die distale Öffnung des Kanals, wo der Faden nach außen in ein Gewebe oder eine Körperhöhle entlassen wird. Da die Vorrichtung das Implantat in fadenförmiger Form und durch den Kanal abgibt, ist eine Applikation in operativer Mikrotechnik möglich.

Vorrichtungen zum Applizieren eines fadenförmigen Implantats durch einen Kanal sind bereits aus dem Stand der Technik bekannt. Zwei Vorrichtungen betreffen genauer spezifizierte fadenförmige Implantatmaterialien. In der US-A-654 564 wird eine Vorrichtung beschrieben, mit der ein mit medizinischen Wirkstoffen behandeltes schnurförmiges Verbandmaterial in den Körper gebracht wird. Die US-A-2 625 934 beschreibt eine Vorrichtung zur Einführung eines weichen Drahtes in einen begrenzten Hohlraum wie beispielsweise ein Aneurysma. Bei beiden Vorrichtungen wird das fadenförmige Implantatmaterial schrittweise mit mechanischen Mitteln durch den Kanal in einen Körperhohlraum vorgeschoben, um diesen mit dem Implantatmaterial zu füllen. Eine weitere Vorrichtung ist in der WO-A-96 04954 beschrieben, welche zur Bildung des Oberbegriffes des Patentanspruchs 1 herangezogen worden ist. Im Kanal dieser Vorrichtung wird ein Fluidstrom erzeugt, mit dem der zu implantierende Faden durch den Kanal transportiert wird. Das Fluid wird dann zusammen mit dem Faden durch die distale Öffnung des Kanals abgegeben. Diese auf der Verwendung eines Fluids basierende Vorrichtung ist weitaus vielseitiger was den Umfang der verwendbaren fadenförmigen Implantationsmaterialien und Kanäle betrifft.

Der Erfindung liegt die Aufgabe zugrunde, eine Vorrichtung zu schaffen, mit deren Hilfe es möglich ist, ein knäuelförmiges Implantat mittels eines Fluids in ein Gewebe oder eine Körperhöhle zu applizieren, wobei das für den Transport des Fadens notwendige Fluid in der Vorrichtung verbleibt. Die Aufgabe ist gemäß Kennzeichen des Anspruchs 1 gelöst.

Die vorliegende Erfindung ermöglicht, den zu implantierenden Faden gegen höhere Widerstände zu injizieren. Da mit der erfindungsgemäßen Vorrichtung im Vergleich zu der Vorrichtung nach der WO-A-96 04954 mehr Kraft auf den Faden ausgeübt werden kann, können auch weniger biegsame Fäden implantiert werden. Zudem erlaubt die erfindungsgemäße Vorrichtung die minimal invasive Implantation fadenförmiger Implantatmaterialien ohne gleichzeitig ein Fluid in den Körper abzugeben. Falls für gewisse medizinische Anwendungen fadenförmige Implantatmaterialien und ein Fluid zusammen implantiert werden sollen, kann dies über zwei verschiedene Kanäle geschehen. Im Gegensatz zu der in der WO-A-96 04954 geschilderten Vorrichtung hat dies den Vorteil, dass das Implantatmaterial und das Fluid getrennt und unabhängig voneinander dosiert werden können. Ebenfalls kann das mittels eines inkompressiblen Fluids implantierte Fadenvolumen genau kontrolliert und dosiert werden, weil das von dem druckerzeugenden Mittel verdrängte Volumen dem implantierten Fadenvolumen entspricht.

Das mittels der erfindungsgemäßen Vorrichtung applizierte Implantat ist durch ein Fadenknäuel in Form einer dreidimensionalen, offenporigen Struktur gekennzeichnet. Dieses Fadenknäuel wird an eine gewünschte Stelle im Gewebe oder eine sonstige Stelle des zu behandelnden Körpers durch einen .kleinen Einstich oder eine Körperöffnung in operativer Mikrotechnik und in beliebiger Menge gebracht. Das Fadenknäuel entsteht, indem der zu applizierende Faden nach Austritt aus dem distalen Ende des Kanals auf einen Widerstand im Gewebe stößt, so dass die nachfolgenden Fadenabschnitte gebogen und schließlich zu dem gewünschten Fadenknäuel abgelegt werden. Durch Bewegungen des distalen Kanalendes während der Implantation eines Fadens kann die Form des Implantats in einem weiten Rahmen variiert und intraoperativ festgelegt werden. Dadurch ergibt sich eine breite Palette möglicher Anwendungen. Die Porengrösse und die Struktureigenschaften des Implantats sind beispielsweise durch den Durchmesser des Fadens oder die Modifikation der Materialeigenschaften des Fadens, insbesondere der Biegesteifigkeit, variierbar und können entsprechend den klinischen Anforderungen eingestellt werden. Der Faden kann Träger von biologisch aktiven Substanzen sein und eignet sich dann insbesondere für die kontrollierte Arzneimittelabgabe oder die Induktion von Körpergewebe.

Die erfindungsgemässe Vorrichtung findet beispielsweise bei der Therapie von Harninkontinenz Verwendung, bei der ein Fadenknäuel in das Gewebe unterhalb des Blasenhalses injiziert wird, um diesen anzuheben. Darüberhinaus findet sie, wie in der WO 96/04954 beschrieben, Anwendung bei der Behandlung von Knochendefekten und in der plastischen Chirurgie, indem gewebeinduzierende Fäden verwendet werden, die zum Beispiel durch die Freisetzung von Wachstumsfaktoren den Aufbau von neuem Knochen-, Knorpel- oder Bindegewebe induzieren. Des weiteren wird die Vorrichtung gemäss der vorliegenden Erfindung zur Behandlung von Fisteln, Aneurismen und zur therapeutischen Embolisation von Blutgefässen verwendet. Ein weiteres wichtiges Anwendungsgebiet ist die Verwendung als "drug delivery system", beispielsweise durch die lokale Abgabe von Zytostatika für die Krebsbehandlung oder durch die Freisetzung von Antibiotika für die Behandlung von Infektionen. Als weiteres Beispiel sei die Zelltransplantation genannt, bei der Fäden mit inkorporierten Zellen, z.B. Langerhanszellen, mit Hilfe der erfindungsgemässen Vorrichtung implantiert werden.

Die hier aufgeführten Beispiele der erfindungsgemässen Vorrichtung stellen nur eine begrenzte Auswahl der verschiedenen Anwendungsmöglichkeiten dar.

Die erfindungsgemäße Vorrichtung zeichnet sich durch eine Öffnung im Bereich des distalen Endes des genannten Kanals aus. Diese Öffnung entspricht im wesentlichen dem Durchmesser oder der Querschnittsfäche des fadenförmigen Implantatmaterials, das auch ein Faden mit unrundem Querschnitt sein kann. Das fadenförmige Implantatmaterial wird durch eine Druckdifferenz durch die Öffnung vorgeschoben, wobei, im Gegensatz zu der aus der WO-A-96 04954 bekannten Ausführung, kein Fluidstrom bewegt wird. Das Fluid wird nur eingesetzt, um den vom druckerzeugenden Mittel aufgebauten hydrostatischen Druck hydraulisch auf den Faden zu Übertragen, der dann dadurch aus der distalen Öffnung des Kanals der Vorrichtung herausgedrückt wird. Bei der erfindungsgemäßen Vorrichtung ist, im Gegensatz zu der aus der WO-A-96 04954 bekannten Vorrichtung, nicht vorgesehen, dass Fluid neben dem Faden aus der Öffnung heraustritt.

Als ein gemäß der vorliegenden Erfindung zu verwendendes Fluid eignet sich ein nicht kompressibler Stoff wie z.B. Wasser oder Öl, da sich die Abgabe des Fadens in das Gewebe besser kontrollieren lässt. Wird der notwendige Druck beispielsweise durch ein kolbenpumpenähnliches System aufgebracht, dann kann bei inkompressiblen Fluiden ein hoher Druck mit sehr wenig Kolbenweg aufgebracht werden, und die Menge des implantierten Materials entspricht dem verdrängten Volumen im Kolben. Inkompressible Fluide haben zudem den Vorteil, dass der Druck sehr schnell und ohne Expansion des Fluids abgebaut werden kann.

Als inkompressible Fluide gemäß der Erfindung eignen sich Wasser, wässrige Lösungen (z.B. Salzlösungen), Öle wie Sojaöl, Rizinusöl oder Paraffin, Flüssigkeiten mit verschiedenen Zusätzen, die das Gleiten des Fadens durch die Öffnung der erfindungsgemäßen Vorrichtung erleichtern und als kompressible Medien Gase wie beispielsweise Stickstoff.

Ein weiterer Vorteil der Erfindung ist darin zu sehen, dass außer biegefesteren auch sehr gut biegeweiche Fäden wie z.B. gummiartige Fäden eingesetzt werden können, die eher zu den gewünschten unregelmäßigen, schwammartigen Knäuelstrukturen führen, die für die meisten Anwendungen in der Medizin besser geeignet sind als regelmäßige Strukturen. Regelmäßige Strukturen können allerdings für verschiedene Anwendungen von Vorteil sein.

Als fadenförmige Implantatmaterialien können Strukturen verwendet werden, die im Vergleich zum Durchmesser eine große Länge aufweisen und die eine genügend kleine Biegesteifigkeit aufweisen, damit sie bei der Implantation zu knäuelartigen Implantatstrukturen geformt werden können. Diese fadenförmigen Strukturen können kreisförmige oder anders geartete Querschnitte aufweisen, Monofilamente oder Multifilamente sein. Wesentlich ist, dass die fadenförmigen Materialien die distale Verengung des Kanals derart abdichten, dass im wesentlichen kein Fluid aus der Vorrichtung dringen kann. Die distale Verengung kann entsprechend den Fadenquerschnitten geformt sein, um einerseits eine gute Dichtung, andererseits gleichzeitig die Passage des Fadens durch die Verengung mit möglichst geringer Reibung zu erreichen.

In besonderen Fällen ist es auch denkbar, biegesteifere Fäden zu verwenden, die im Körper keine Knäuelstrukturen ausbilden können, z.B. um mittels der erfindungsgemäßen Vorrichtung einen Faden im Gewebe linear vorzuschieben oder geordnete Strukturen, beispielsweise in der Form von Spiralfedern, zu applizieren.

Als Materialien für das fadenförmige Implantat eignen sich synthetische Polymere wie z.B. Silikonelastomere, Polyhydroximethacrylat (PHEMA) und Hydrogele aus PHEMA, Polyurethane, Dacron, Polyester wie Polylactide oder Polyglycolide und Polyorthoester. Als Biopolymere eignen sich zum Beispiel Collagen, Gelatine, Hyaluronsäure, Chitosan und Alginat. Des weiteren können Verbundmaterialien wie Monofilamente mit Faserverstärkung aus Glasfasern, Polymerfasern oder Keramikfasern, sowie Monofilamente mit mechanischer Verstärkung durch eine oder mehrere lange Fasern oder eines Multifilaments, Monofilamente mit inkorporierten Partikeln, Monofilamente aus verschiedenen Werkstoffen (resorbierbar oder nicht resorbierbar) beispielsweise mit schichtartigem Aufbau, poröse Monofilamente, Monofilamente, die im Körper aufquellen (quellbares PHEMA oder andere Hydrogele), Monofilamete mit Röntgenkontrast (auch durch Inkorporation von Partikeln oder Fasern oder Fäden mit Röntgenkontrast) verwendet werden.

Die Injektion von Fäden mittels den aus dem Stand der Technik bekannten Verfahren gestaltet sich unter Umständen schwierig, wenn der Raum für das Implantat im Gewebe während der Injektion geschaffen werden muss, wie das zum Beispiel beim sogenannten "tissue bulking" der Fall ist. Die Kraft, die hierbei auf den Faden ausgeübt werden muss, um ihn in das umgebende Gewebe abzugeben, ist verhältnismässig gross. Gemäss dem in der W096/04954 beschriebenen Verfahren gerät der Faden während des Implantiervorganges häufig ins Stocken, da eine verhältnismässig geringe Kraft aufgebracht wird, mit der der Faden gegen den Widerstand des Gewebes im Kanal vorgeschoben wird. Das Vorschieben des Fadens geschieht durch das in der WO 96/04954 verwendete Fluid, das mit einem entsprechenden Druck eingebracht werden kann. Die Kraft auf den Faden kann nur bewirken, dass der von dem Fluid geschaffene Hohlraum mit dem Fadenmaterial ausgefüllt wird. Die Struktur des Implantats wird daher sehr locker sein und sich erst mit der Resorption bzw. mit dem Absaugen des Fluids verkleinern beziehungweise verdichten. Bei dem obengenannten "tissue bulking" ist eine gewisse Größe des Implantats erforderlich. Häufig ist es nicht möglich, den Raum für das Implantat zunächst durch Überdehnen zu schaffen, um dann später auf das gewünschte Volumen zu kommen. Außerdem dürfte es sehr schwierig sein, unter diesen Umständen die notwendige Fadenmenge, d.h. das Implantatvolumen zu dosieren.

Ein weiterer Vorteil des vorliegenden Verfahrens ist daher darin zu sehen, dass der Faden direkt in das Gewebe gedrückt werden kann und allenfalls eine nur geringe Verkleinerung des Implantatvolumens durch Umlagerung der Knäuelstruktur zu erwarten ist. Darüber hinaus ist es möglich, weiche und im Vergleich zur WO-A-96 04954 biegesteifere Fäden zu verwenden, die sich mit der in der WO-A-96 04954 beschriebenen Vorrichtung nicht applizieren lassen.

Je nach klinischer Anwendung kann es sinnvoll sein, einen oder mehrere Fäden mit vorbestimmter Länge zu applizieren oder die Applikation nach der Implantation einer aureichenden Menge des fadenförmigen Implantatmaterials abzubrechen. Im ersten Fall wird mit dem Verschluss der distalen Öffnung oder mit dem Einsatz eines Absperrventils zwischen druckerzeugendem Mittel und Gehäuse der plötzliche Austritt von unter Druck stehendem Fluid nach dem Ausstoß des proximalen Fadenendes verhindert. Im zweiten Fall wird der Faden in der Nähe des distalen Endes des Kanals oder weiter proximal nach der Applikation von genügend Implantatmaterial durchtrennt. Der distale Teil des durchtrennten Fadens kann dann allenfalls noch vollständig aus der Vorrichtung ausgestoßen werden, bevor der Kanal aus dem behandelten Körper zurückgezogen wird.

Weitere Merkmale und Vorteile der vorliegenden Erfindung ergeben sich aus den abhängigen Anprüchen, der Beschreibung und den Figuren. Ausführungsbeispiele der Erfindung werden nachfolgend anhand der Figuren erläutert:
- Figur 1:: schematische Ansicht einer erfindungsgemässen Vorrichtung
- Figur 2a:: Lippendichtung
- Figur 2b:: Dichtungseinsatz
- Figur 2c:: Dichtung durch Lochscheibe
- Figur 2d:: Dichtungseinsatz und umgebogene Enden des Kanals
- Figur 2e:: Dichtung durch Verengung am Kanalende
- Figur 2f:: Dichtung durch Verengung des Kanals mit abgeschrägter Kanalspitze
- Figur 2g:: Konischer Dichtungseinsatz
- Figur 2h:: Seitlicher Ausgang für den Faden
- Figur 2i:: Dichtung durch O-Ring
- Figur 3a:: Abschervorrichtung in der Nähe des Fadenspeichers
- Figur 3b:: Variante mit Bolzen und offenem Durchgang
- Figur 3c:: Variante mit Bolzen, ohne offenen Durchgang
- Figur 4a:: Abschervorrichtung an der Spitze des Kanals
- Figur 4b:: Abschervorrichtung mit gebogenem Metallplättchen mit Loch
- Figur 4c:: Abschneiden das Fadens mittels eines Drahts
- Figur 4d:: Durchtrennen des Fadens beim Zurückziehen von proximal
- Figur 5a:: Variante mit einer Kugel
- Figur 5b:: Variante mit drehbar gelagertem Plättchen
- Figur 5c:: Ventilverschluss
- Figur 6:: Gestaltung des distalen Fadenendes

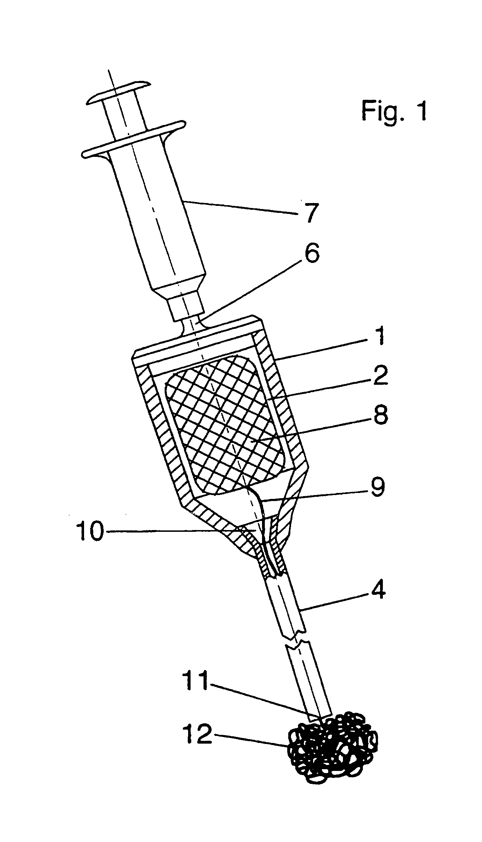

Die erfindungsgemässe Vorrichtung weist gemäss Figur 1 ein Gehäuse 1 auf, welches einen Innenraum 2 besitzt, der in einen Kanal 4 mündet und der über einen weiteren Kanal 6 beispielsweise mit einer spritzenähnlichen Pumpvorrichtung 7 verbunden ist. Der Innenraum der Vorrichtung (2, 7, 4) ist mit einem Fluid gefüllt.

Gemäss einer anderen, hier nicht dargestellten Ausführung kann die Pumpvorrichtung 7 auch direkt an den Kanal 4 unter Umgehung des Gehäuses 1 angeschlossen sein.

Im Innenraum 2 befindet sich ein zu einer Bobine 8 gewickelter Faden, der beispielsweise von innen über Kopf von der Bobine abgezogen werden kann. In einer weiteren Ausführungsform der vorliegenden Erfindung kann der Faden 9 auch zu einer Spule aufgewickelt und auf einer drehbaren Achse im Gehäuse 1 untergebracht sein oder in Form von weiteren, hier nicht gezeigten Vorratsmöglichkeiten. Ein auf der Spule bzw. der Bobine aufgewickelter Faden 9 ist durch eine proximale Öffnung 10 des Kanals 4, den Kanal 4 und die distale Öffnung 11 des Kanals 4 eingezogen. Der Kanal 4 ist so ausgebildet, dass der Faden 9 im wesentlichen ohne Reibung in ihm gleiten kann. Im Bereich der distalen Öffnung 11 ist der Kanal 4 derart verengt, dass der Faden 9 durch die Öffnung 11 nach aussen abgegeben werden kann, gleichzeitig jedoch das Fluid in der Vorrichtung zurückbehalten wird. Der Faden 9 wird durch einen durch die Pumpvorrichtung 7 erzeugten hydrostatischen Druck im Inneren der Vorrichtung durch die distale Öffnung 11 nach aussen gedrückt. Je nach Höhe des Druckes im Fluid kann die Transportgeschwindigkeit des Fadens 9 erhöht oder verlangsamt werden. Auf diese Weise ist es möglich, eine zu bestimmende Fadenlänge durch die distale, verengte Öffnung 11 unter Abwicklung von beispielsweise einer Bobine nach aussen abzugeben, ohne dass gleichzeitig Fluid aus der Vorrichtung austritt.

Der Kanal 4 ist so ausgebildet, dass seine distale Öffnung 11 durch eine Einstichöffnung oder Körperöffnung in das Gewebe der zu behandelnde Körperstelle oder in einen Hohlraum des Körpers vorschiebbar ist. Der Kanal kann beispielsweise als biegbarer Katheter, starrer oder biegbarer Endoskopkanal, als Einsatz in einen Endoskopkanal oder Trokar oder als Hohlnadel ausgeführt sein. Das an der Öffnung 11 austretende distale Fadenende erfährt im Gewebe einen Widerstand, so dass die nachfolgenden Fadenabschnitte gebogen und schliesslich in einem Fadenknäuel 12 abgelegt werden, wie Figur 1 zeigt. Dies ist typischerweise für die in WO 96/04954 beschriebene Vorrichtung wichtig.

Ein wesentliches Merkmal der vorliegenden Erfindung liegt darin, dass im wesentlichen nur der Faden 9 aus der distalen Öffnung austritt und das Fluid mittels spezieller Ausführungsformen der distalen Öffnung zurückgehalten wird. Diese speziellen Ausführungsformen werden im folgenden beschrieben:

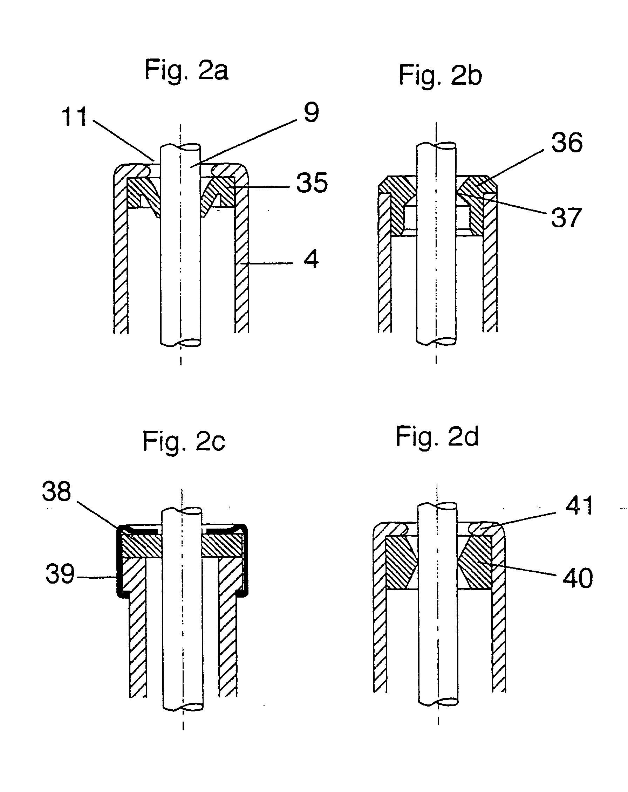

Figur 2a zeigt eine Lippendichtung 35, die den Kanal 4 an der distalen Öffnung 11 verschliesst.
Figur 2b stellt einen Einsatz 36 dar, der den Kanal 4 über die Kante 37 abdichtet und durch Presssitz, Verschweissen und Kleben montiert wird. Er dient als Aufsatz sowohl für Kanäle aus Metallen als auch harten und weichen Kanälen aus Polymeren.
Figur 2c zeigt eine Dichtung mittels einer Lochscheibe 38. Die Lochscheibe 38 kann aus harten oder weichen Materialien wie zum Beispiel Gummi bestehen. In der hier gezeigten Ausführungsform erfolgt die Fixierung der Scheibe 38 mit einer Kappe 39 aus Metall, wobei die Montage der Lochscheibe 38 unter plastischer Deformation der Kappe 39 erfolgt.
In Figur 2d ist ein Dichtungseinsatz 40 aus hartem oder weichem Material dargestellt, der durch die umgebogenen Kanten 41 des Kanals fixiert wird. Eine weitere Möglichkeit der Fixierung des Dichtungseinsatzes 40 besteht z.B. im Aufbringen einer Verengung des Kanals.

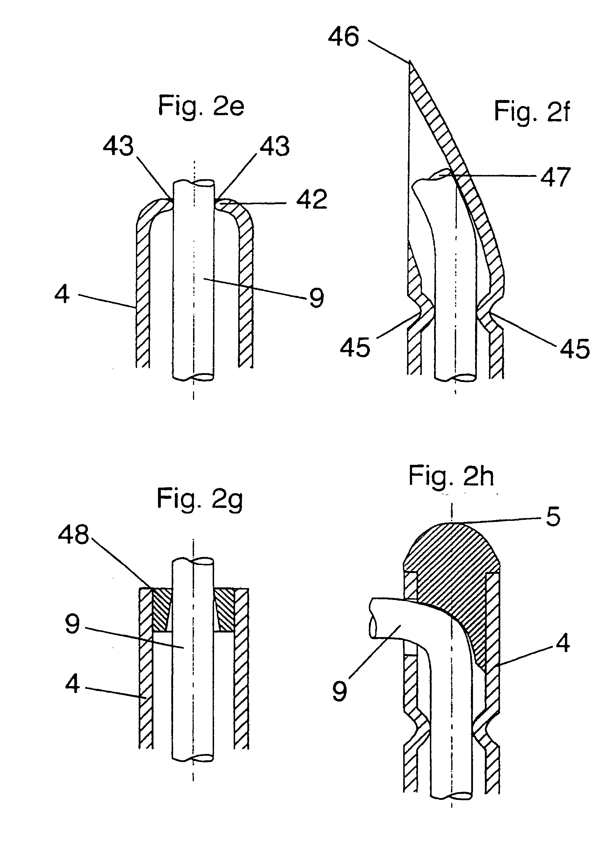

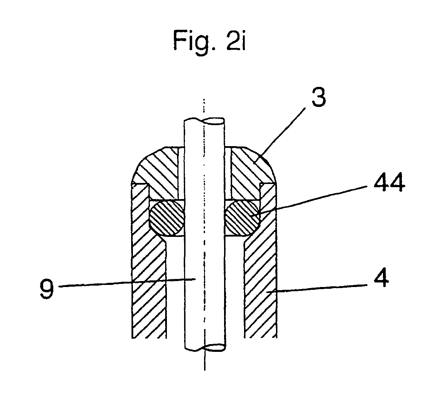

Figur 2e zeigt eine Verengung 42 am Ende eines beispielsweise metallischen Kanals 4, dessen Kante 43 mit speziellen Werkzeugen wie dargestellt verformt wird, wobei die Kante 43 auch abgerundet sein kann, um die Gleitreibung des Fadens 9 beim Vorschub durch die Verengung 42 zu reduzieren.
Figur 2f veranschaulicht eine Kanalausführung, bei der die Dichtung durch eine Verengung 45 des Kanals 4 bewirkt wird. Der Kanal 4 ist mit einer gebogenen und geschliffenen Spitze 46 ausgebildet, um das Vorschieben in das Gewebe zu erleichtern. Diese Ausführungsform hat den Vorteil, dass das distale Fadenende 47 während dem Vorschub geschützt ist und nicht von dem Gewebe durch die Verengung 45 zurückgestossen werden kann. In einer weiteren, hier nicht dargestellten Ausführungsform der Figur 2f, kann die Spitze auch gerade ausgebildet sein.
Figur 2g veranschaulicht einen konischen Dichtungseinsatz 48, der durch Kleben, Verschweissen oder Presssitz fixiert wird. In einer weiteren Ausführungsform ist der Konus nicht wie dargestellt nach innen, sondern nach aussen orientiert.
Figur 2h zeigt eine Variante, bei der der Faden 9 seitlich aus der Kanal 4 ausgeführt wird. In einer weiteren Ausführungsform kann der Fadenausgang auch vom distalen Ende 5 des Kanals 4 weiter entfernt sein.
Figur 2i zeigt eine Ausführung, bei der der Kanal 4 mit einem O-Ring gedichtet wird. Der O-Ring 44 ist bei der dargestellten Ausführung mit einem Einsatz 3 fixiert, der durch Kleben, Verschweissen oder Presssitz montiert wird.

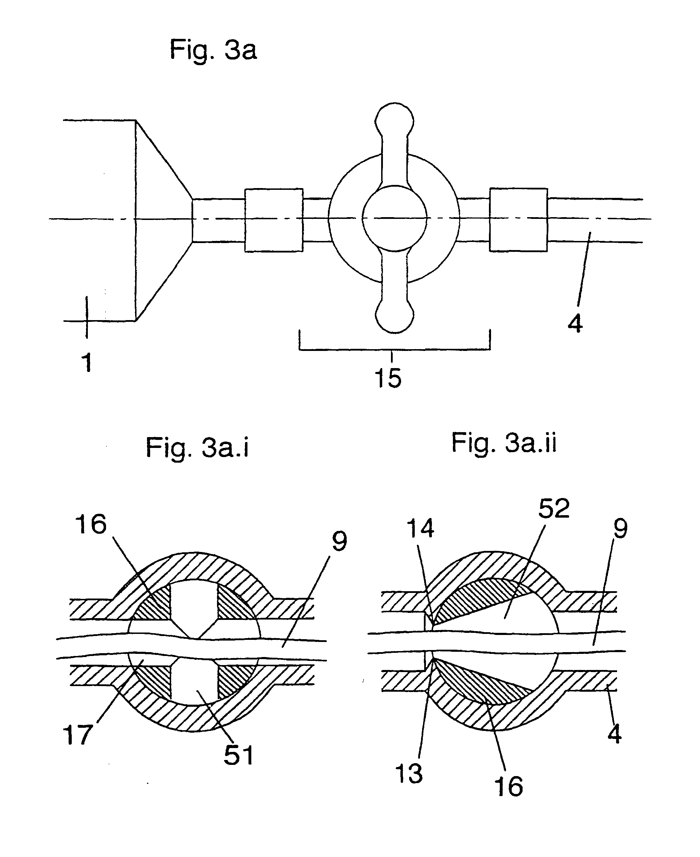

Figur 3a zeigt als weitere Ausführungsform der vorliegenden Erfindung eine Abschervorrichtung 15 für den Faden 9 proximal der distalen Öffnung 11 des Kanals, die manuell bedienbar ist. Die Abschervorrichtung 15 ist identisch oder vergleichbar mit herkömmlichen Ventilen, z.B. einem Dreiweghahn. Figuren 3a.i und 3a.ii zeigen einen schematischen Schnitt durch verschiedene Ausführungsformen der Abschervorrichtung 15. In Figur 3a.i wird der in einer Bohrung 17 verlaufende Faden 9 durch eine Drehung des Zylinders 16 um 90° am Zylindergehäuse abgeschert. Danach verbleibt wegen der zweiten Bohrung 51 ein offener Kanal, um den distalen Teil des Fadens zu applizieren.
Figur 3a.ii zeigt eine Abscherung an scharfen Kanten 13 und 14. Der Faden 9 verläuft in der konischen Bohrung 52 im Zylinder 16. Das Abscheren erfolgt durch Drehen des Zylinders 16 um ca. 45°: Bei der Ausführungsform 3a.ii bleibt ein durchgängiger Kanal nach Zurückdrehen des Zylinders erhalten, um die Applikation des distalen Teils des in dem Kanal 4 verbleibenden Fadens 9 zu ermöglichen. Je nach Materialeigenschaften des Fadens 9 ist das Abscheren an stumpfen oder scharfen Kanten vorteilhaft.

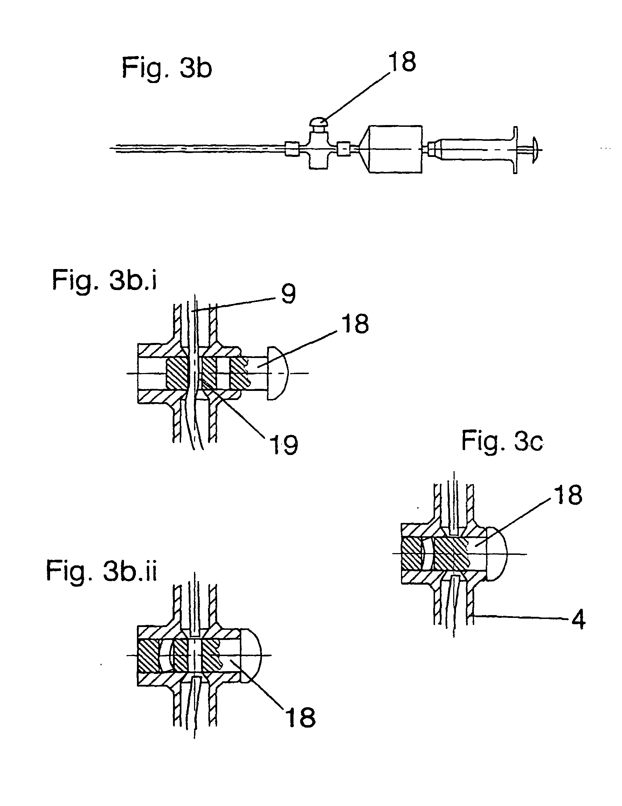

Figur 3b zeigt eine weitere Ausführungsform der Abschervorrichtung, bei der ein Bolzen 18 mit zwei Querbohrungen, wobei durch eine davon (19) der Faden vor und während der Applikation geführt wird, seitlich in den Kanal gedrückt wird und beim Hineindrücken den Faden 9 abschert. Auch bei dieser Abschervorrichtung verbleibt wegen der zweiten Querbohrung ein durchgängiger Kanal nach dem Abscheren.
Figur 3c zeigt eine weitere Ausführung der Abschervorrichtung gemäss Figur 3b mit einem Bolzen 18 mit nur einer Querbohrung. Bei dieser Ausführung ist nach dem Abscheren des Fadens durch Hineindrücken des Bolzens 18 der Kanal 4 verschlossen. Diese Variante ist beispielsweise anwendbar, wenn das drukkerzeugende Mittel 7 unter Umgehung des Gehäuses 1 distal der Abschervorrichtung direkt in den Kanal 4 eingeleitet wird.

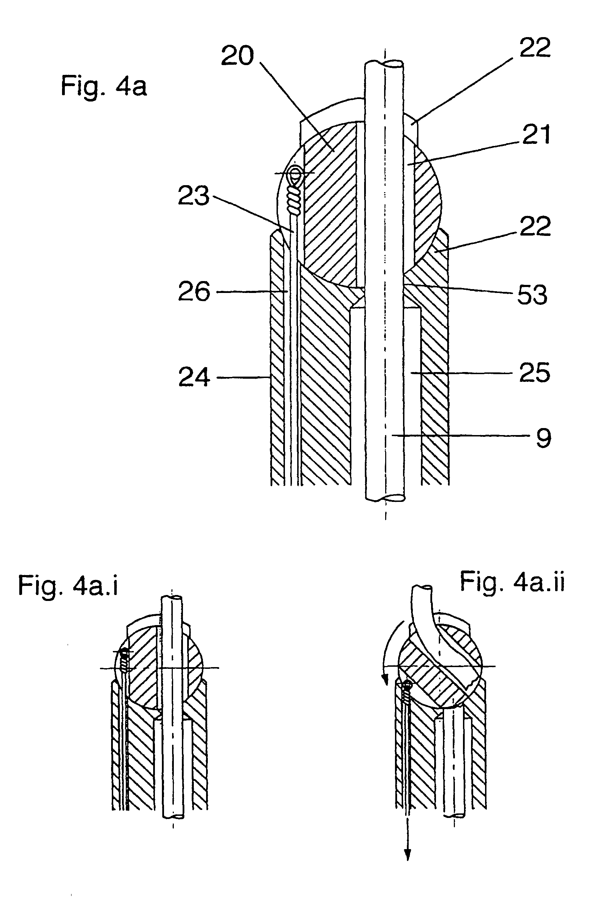

Figuren 4a bis 4d zeigen verschiedene Abschervorrichtungen an der distalen Öffnung 11 der erfindungsgemässen Vorrichtung, d.h. am Ende des Kanals 4.
Figur 4a veranschaulicht ein ähnliches System wie für Figur 3a beschrieben. Ein zylindrischer Einsatz 20 mit Bohrung 21 ist in einer Halterung 22 drehbar eingeschnappt. Über einen Zugdraht 23 kann der zylindrische Einsatz 20 rotiert werden, wodurch der Faden 9 abgeschert und das Lumen 25 gleichzeitig verschlossen wird (Figuren 4a.i und 4a.ii). Vorzugsweise werden doppellumige Kanäle 24 verwendet, wobei der Zugdraht 23 durch Lumen 26 und der Faden 9 durch Lumen 25 läuft. Die Figuren 4a.i und 4a.ii zeigen schematisch die Drehbewegung des zylindrischen Einsatzes 20. Diese Ausführung enthält auch gleichzeitig eine distale Verengung 53 des Lumens 25 um das Lumen 25 während der Applikation abzudichten.

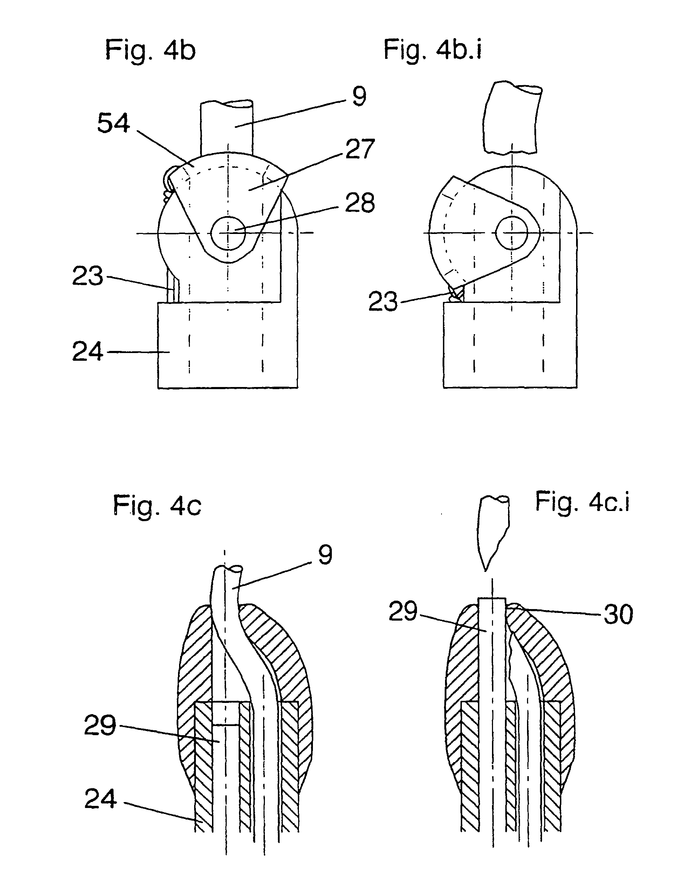

Figur 4b zeigt ein ähnliches, wie in Figur 4a beschriebenes System zum Abscheren des Fadens 9. Hier befindet sich an der distalen Öffnung 11 des Kanals 4 ein um die Achse 28 drehbar gelagertes, gebogenes Metallplättchen 27 mit einem Loch 54, an dessen Kanten der Faden abgeschert wird (Fig. 4b.i). Das Plättchen wird dabei über einen Zugdraht 23 bewegt. Der Kanal wird bei bei der hier dargestellten Ausführungsform nicht verschlossen.

Figur 4c zeigt eine Abschervorrichtung, bei der das Mittel zum Abscheren des Fadens 9 ein Draht 29 ist. Der Faden 9 und der Draht 29 verlaufen in getrennten Lumen des doppellumigen Kanals 24, wobei sich die Volumen am distalen Ende des Kanals 24 vereinen. Die Spitze des Kanals ist asymmetrisch gestaltet, um eine gradlinige Führung des Drahts zu gewährleisten. Nach dem Abscheren des Fadens ist die distale Verengung 30 durch den Draht 29 verschlossen.

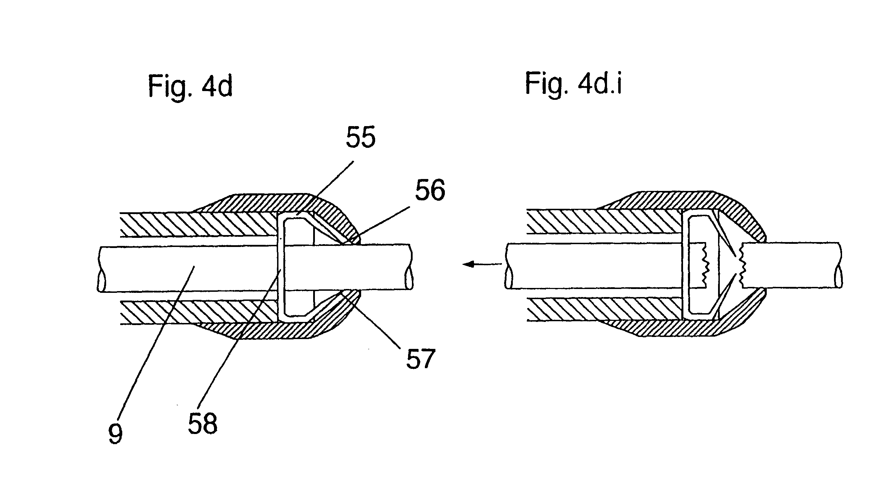

Figur 4d stellt eine Abschervorrichtung dar, bei der der Faden 9 beim Zurückziehen nach proximal durchtrennt wird. In der dargestellten Ausführung erfolgt dies mittels eines gebogenen Plättchens 55 mit scharfen Kanten 56 und 57 und einem Loch 58, durch welches der Faden 9 während der Applikation geleitet wird. Die Kanten 56 und 57 stehen mit dem Faden 9 in Kontakt. Beim Zurückziehen des Fadens nach proximal (Figur 4d.i) schneiden sich die Kanten 56 und 57 in den Faden 9. Der Faden 9 wird ganz durchschnitten oder reisst an der geschwächten Stelle. Das Zurückziehen des Fadens kann beispielsweise dadurch erfolgen, dass das Gehäuse 1 vom Kanal 4 getrennt wird und am Faden 9 von Hand gezogen wird.

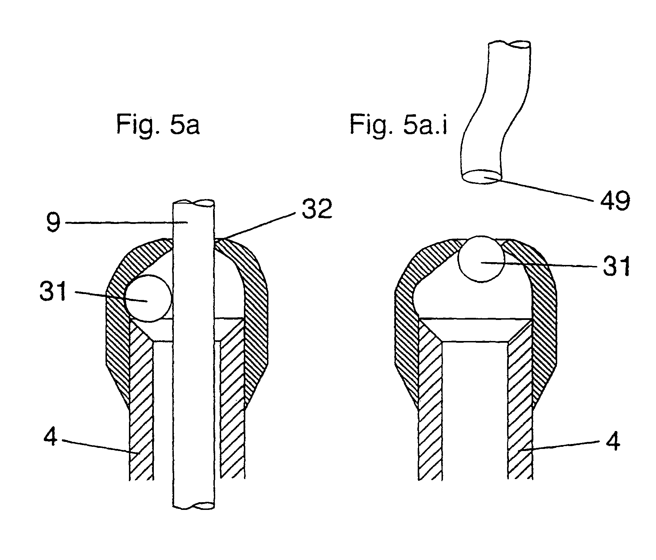

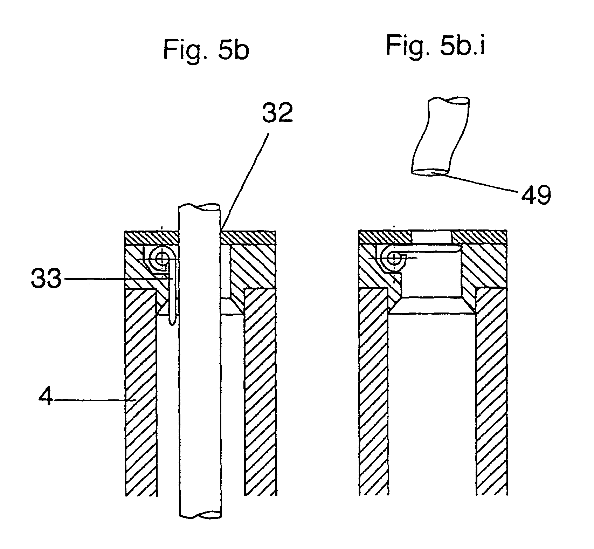

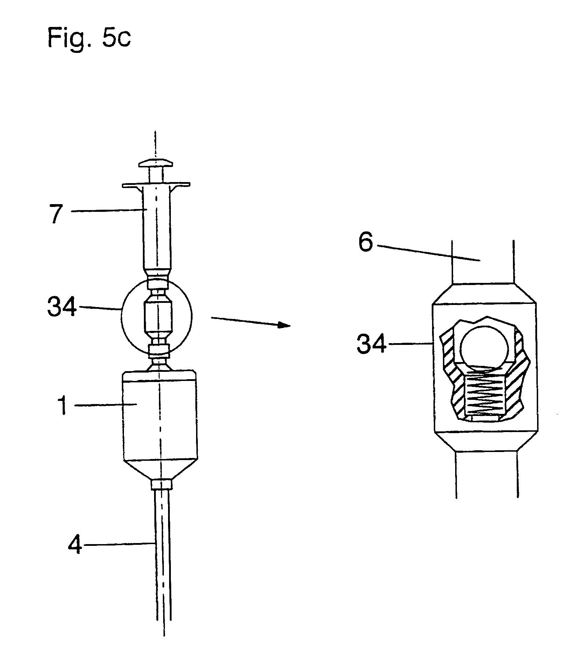

Figuren 5 zeigen verschiedene Verschlussvorrichtungen für den Kanal 4. Je nach verwendetem Fluid und klinischer Applikation ist ist es wünschenswert, dass kein oder nur sehr wenig Fluid mit dem Faden 9 in den Körper eingebracht wird. Nach Applikation einer definierten Fadenlänge ist ein automatisches Verschliessen des Kanals 4 erforderlich, um zu verhindern, dass das Fluid mit erhöhtem Druck aus der distalen Öffnung 32 des Kanals 4 austritt, nachdem diese nicht mehr durch den Faden 9 verschlossen ist.

Figur 5a zeigt eine Ausführungsform des Verschlusses des Kanals 4 durch eine Kugel 31, die, wie in Figur 5a.i gezeigt, nach dem Austritt des proximalen Fadenendes 49 aus dem Kanal 4 durch das Fluid auf die distale Verengung 32 des Kanals verschoben wird und diesen dadurch verschließt.

Figur 5b zeigt eine Ausführungsform mit einem drehbar gelagerten Plättchen 33, das zuklappt und die Öffnung des Kanals 4 verschließt, wenn das proximale Fadenende 49 die Verengung 32 passiert hat. Die Kraft zum Verschließen der Öffnung kann entweder wie hier dargestellt durch das Fluid aufgebracht werden oder in einer anderen, hier nicht dargestellten Variante durch eine Feder, die das Plättchen bewegen kann.

Figur 5c stellt ein Absperrventil 34 dar, das zwischen der spritzenähnlichen Pumpvorrichtung 7 und dem die Bobine 8 umschließenden Gehäuse 1 geschaltet ist. Das Ventil 34 schließt bei plötzlich höherem Fluiddurchsatz, wie dies beim Austritt des proximalen Fadenendes durch die distale Öffnung des Kanals 4 auftritt, den Kanal 6 zwischen der Pumpvorrichtung 7 und dem Gehäuse 1 und stoppt somit die Injektion von Fluid.

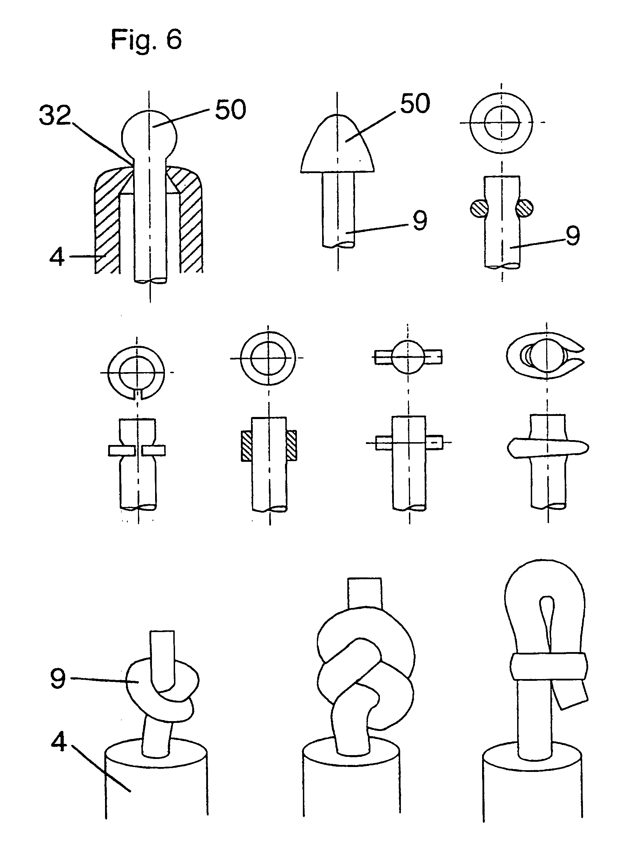

Um ein Durchschlüpfen des distalen Fadenendes 50 durch die Verengung 32 an der Spitze des Kanals 4 während des Vorschiebens der erfindungsgemäßen Vorrichtung im zu behandelnden Gewebe zu verhindern, werden Verdickungen am Fadenende angebracht. Verschiedene Möglichkeiten der Gestaltung des Fadenendes sind in Figur 6 dargestellt.

## Patentansprüche

1. Vorrichtung zum Applizieren eines fadenförmigen Implantatmaterials mit einem druckerzeugenden, ein Fluid enthaltenden Mittel, das mit einem Gehäuse (1) in Verbindung steht, welches in einen Kanal (4) mit einer distalen Öffnung (11) mündet, durch die das sich im Gehäuse (1) befindende und sich durch den Kanal (4) erstreckende fadenförmige Material mittels des druckerzeugenden Mittels und des Fluids aus der Vorrichtung transportiert wird, **dadurch gekennzeichnet, dass** die distale Öffnung (11) eine Verengung umfasst, durch welche das fadenförmige Material passieren kann und die das fadenförmige Material derart umschließt, dass das Fluid in der Vorrichtung verbleibt, wobei das fadenförmige Material durch die Verengung bewegt wird, indem das druckerzeugende Mittel mittels des Fluids im Innenraum der Vorrichtung einen statischen Druck erzeugt.

2. Vorrichtung gemäß Anspruch 1, **dadurch gekennzeichnet, dass** das distale Ende des Kanals (4) mit einer den Austritt des Fluids aus dem Kanal verhindernden Dichtung versehen ist.

3. Vorrichtung gemäß Anspruch 2, **dadurch gekennzeichnet, dass** die Dichtung in Form einer Lippendichtung (35) ausgebildet ist.

4. Vorrichtung gemäß Anspruch 2, **dadurch gekennzeichnet, dass** die Dichtung aus einem Einsatz (36) besteht.

5. Vorrichtung gemäß Anspruch 4, **dadurch gekennzeichnet, dass** der Einsatz (36) durch Presssitz, Verschweißen, Formschluss oder umgebogene Kanten angebracht wird.

6. Vorrichtung gemäß Anspruch 2, 4 oder 5, **dadurch gekennzeichnet, dass** die Dichtung aus einer Lochscheibe (38) besteht.

7. Vorrichtung gemäß Anspruch 2, **dadurch gekennzeichnet, dass** die Dichtung (42) aus einer Verjüngung des Kanals (4) im Bereich des distalen Endes (11) des Kanals (4) besteht.

8. Vorrichtung gemäß Anspruch 7, **dadurch gekennzeichnet, dass** eine Kante (43) des Kanals zum Kanalinnenraum hin verformt ist.

9. Vorrichtung gemäß Anspruch 2, **dadurch gekennzeichnet, dass** die Dichtung (45) durch eine Verengung des Kanals (4) proximal der distalen Öffnung (11) des Kanals gebildet wird.

10. Vorrichtung gemäß Anspruch 2, 4 oder 5, **dadurch gekennzeichnet, dass** die Dichtung als ein sich nach außen verengender konischer Einsatz (48) ausgebildet ist.

11. Vorrichtung gemäß Anspruch 2 oder 4, **dadurch gekennzeichnet, dass** die Dichtung als O-Ring ausgebildet ist.

12. Vorrichtung gemäß einem der Ansprüche 1-11, **dadurch gekennzeichnet, dass** die distale Öffnung (11) des Kanals (4) zu einer Spitze (46) angeschrägt ist.

13. Vorrichtung gemäß einem der Ansprüche 1-12, **dadurch gekennzeichnet, dass** sich proximal der Öffnung (11) des Kanals (4) ein Mittel zum Durchtrennen des Fadens befindet.

14. Vorrichtung gemäß Anspruch 13, **dadurch gekennzeichnet, dass** das Mittel zum Durchtrennen des Fadens (9) einen Zylinder (16) mit einer den Faden führenden Bohrung (17, 52) umfasst, durch dessen Drehung der Faden (9) am Zylindergehäuse abgeschert wird.

15. Vorrichtung gemäß Anspruch 13, **dadurch gekennzeichnet, dass** die Durchtrennung des Fadens (9) mit einem Bolzen (18) erfolgt, der seitlich in den Kanal (4) hineingeschoben wird.

16. Vorrichtung gemäß einem der Ansprüche 1-12, **dadurch gekennzeichnet, dass** sich ein Mittel zum Durchtrennen des Fadens (9) im Bereich der distalen Öffnung (11) des Kanals (4) befindet.

17. Vorrichtung gemäß Anspruch 16, **dadurch gekennzeichnet, dass** das Mittel zum Durchtrennen des Fadens (9) einen zylindrischen Einsatz (20) mit einer Bohrung (21) umfasst, welcher mittels eines Zugdrahts -(23) gedreht werden kann.

18. Vorrichtung gemäß Ansprüchen 16, **dadurch gekennzeichnet, dass** das Mittel zum Durchtrennen des Fadens (9) ein Plättchen (27) mit einem Loch (54) umfasst, welches mittels eines Zugdrahts (23) bewegt werden kann.

19. Vorrichtung gemäß Anspruch 16, 17 oder 18, **dadurch gekennzeichnet, dass** ein doppellumiger Kanal (24) verwendet wird, wobei der Zugdraht (23) durch das eine Lumen (26) und der Faden (9) durch das andere Lumen (25) läuft.

20. Vorrichtung gemäß Anspruch 16, **dadurch gekennzeichnet, dass** das Mittel zum Durchtrennen des Fadens (9) ein Draht (29) ist und das Durchtrennen des Fadens (9) durch das Vorschieben des Drahts (29) und mit Hilfe des distalen Endes (59) des Drahts (29) erfolgt.

21. Vorrichtung gemäß einem der Ansprüche 16 bis 20, **dadurch gekennzeichnet, dass** gleichzeitig mit dem Durchtrennen des Fadens (9) der Kanal (4) verschlossen wird.

22. Vorrichtung gemäß einem der Ansprüche 16 bis 20, **dadurch gekennzeichnet, dass** eine oder mehreren scharfen Kanten mit geeigneten Mitteln so angebracht sind, dass der Faden durchgetrennt wird, wenn er nach proximal durch den Kanal (4) zurückgezogen wird.

23. Vorrichtung gemäß Anspruch 15 oder 21, **dadurch gekennzeichnet, dass** das Mittel zum Durchtrennen des Fadens (9) ein gebogenes Plättchen (55) umfasst, welches an seiner der Öffnung zugewandten Seite zwei mit dem Faden in Kontakt stehende scharfe Kanten aufweist.

24. Vorrichtung gemäß einem der Ansprüche 1-23, **dadurch gekennzeichnet, dass** sich am distalen Ende des Kanals (4) ein Verschlussmittel befindet, welches nach Austritt des proximalen Fadenendes (49) aus dem Kanal (4) denselben unter Zurückhaltung des sich in dem Kanal (4) befindlichen Fluids veschließt.

25. Vorrichtung gemäß Anspruch 24, **dadurch gekennzeichnet, dass** die distale Verengung des Kanals (4) mittels einer Kugel (31) verschlossen wird.

26. Vorrichtung gemäß Anspruch 24, **dadurch gekennzeichnet, dass** das Verschlussmittel ein drehbar gelagertes Plättchen (33) umfasst, welches nach Austritt des proximalen Fadenendes (49) in Richtung der distalen Öffnung (11) des Kanals (4) klappt und diesen verschließt.

27. Vorrichtung gemäß einem der Ansprüche 1 bis 26, **dadurch gekennzeichnet, dass** ein Absperrventil (34) nach dem druckerzeugenden Mittel geschaltet ist, welches nach Austritt des proximalen Fadenendes (49) aus dem Kanal (4) aufgrund des Anstieges des Fluidstroms schließt und damit ein Einspritzen von Fluid in den Körper verhindert.

## Claims

1. Device for applying a thread-shaped implant material with a pressure-generating means which contains a fluid and communicates with a housing (1) which leads into a duct (4) with a distal opening (11) through which the thread-shaped material, which is located in the housing (1) and extends through the duct (4), is transported out of the device by means of the pressure-generating means and the fluid, **characterised in that** the distal opening (11) comprises a contraction through which the thread-shaped material can pass and which encloses the thread-shaped material such that the fluid remains in the device, wherein the thread-shaped material is moved through the contraction as a result of the pressure-generating means generating a static pressure by means of the fluid in the interior of the device.

2. Device according to Claim 1, **characterised in that** the distal end of the duct (4) is provided with a seal which prevents the fluid from escaping from the duct.

3. Device according to Claim 2, **characterised in that** the seal is in the form of a lip seal (35).

4. Device according to Claim 2, **characterised in that** the seal consists of an insert (36).

5. Device according to Claim 4, **characterised in that** the insert (36) is fitted by a press fit, welding, a positive fit or bent-over edges.

6. Device according to Claim 2, 4 or 5, **characterised in that** the seal consists of a perforated disc (38).

7. Device according to Claim 2, **characterised in that** the seal (42) consists of a taper of the duct (4) in the region of the distal end (11) of the duct (4).

8. Device according to Claim 7, **characterised in that** one edge (43) of the duct is shaped towards the duct interior.

9. Device according to Claim 2, **characterised in that** the seal (45) is formed by a contraction of the duct (4) in the proximity of the distal opening (11) of the duct.

10. Device according to Claim 2, 4 or 5, **characterised in that** the seal is formed as a conical insert (48) which narrows outwards.

11. Device according to Claim 2 or 4, **characterised in that** the seal is formed as an 0-ring.

12. Device according to any one of Claims 1 - 11, **characterised in that** the distal opening (11) of the duct (4) is bevelled to a point (46).

13. Device according to any one of Claims 1 - 12, **characterised in that** a means for severing the thread is located in the proximity of the opening (11) of the duct (4).

14. Device according to Claim 13, **characterised in that** the means for severing the thread (9) comprises a cylinder (16) with a bore (17, 52) which guides the thread, the rotation of which cylinder shears off the thread (9) at the cylinder housing.

15. Device according to Claim 13, **characterised in that** the thread (9) is severed by a pin (18) which is pushed into the duct (4) at the side.

16. Device according to any one of Claims 1 - 12, **characterised in that** a means for severing the thread (9) is located in the region of the distal opening (11) of the duct (4).

17. Device according to Claim 16, **characterised in that** the means for severing the thread (9) comprises a cylindrical insert (20) with a bore (21), which insert can be rotated by means of a pull wire (23).

18. Device according to Claim 16, **characterised in that** the means for severing the thread (9) comprises a lamina (27) with a hole (54), which lamina can be moved by means of a pull wire (23).

19. Device according to Claim 16, 17 or 18, **characterised in that** a double-lumen duct (24) is used, wherein the pull wire (23) runs through one lumen (26) and the thread (9) runs through the other lumen (25).

20. Device according to Claim 16, **characterised in that** the means for severing the thread (9) is a wire (29), and the thread (9) is severed by advancing the wire (29) and by means of the distal end (59) of the wire (29).

21. Device according to any one of Claims 16 - 20, **characterised in that** the duct (4) is closed at the same time as the thread (9) is severed.

22. Device according to any one of Claims 16 - 20, **characterised in that** one or a plurality of sharp edge(s) is/are fitted with suitable means such that the thread is severed when it is withdrawn proximally through the duct (4).

23. Device according to Claim 15 or 21, **characterised in that** the means for severing the thread (9) comprises a bent lamina (55) which, at its side which faces the opening, comprises two sharp edges which are in contact with the thread.

24. Device according to any one of Claims 1 - 23, **characterised in that** a closing means is located at the distal end of the duct (4), which means closes the duct (4) after the proximal thread end (49) has emerged from the latter while holding back the fluid located in the duct (4).

25. Device according to Claim 24, **characterised in that** the distal contraction of the duct (4) is closed by means of a ball (31).

26. Device according to Claim 24, **characterised in that** the closing means comprises a rotatably mounted lamina (33) which, after the proximal thread end (49) has emerged, shuts in the direction of the distal opening (11) of the duct (4) and closes this.

27. Device according to any one of Claims 1 to 26, **characterised in that** a shutoff valve (34) is connected after the pressure-generating means, which valve, after the proximal thread end (49) has emerged from the duct (4), closes on account of the increase in the fluid flow and thus prevents fluid from being injected into the body.

## Revendications

1. Dispositif d'application d'une matière d'implantation filiforme avec un agent générant une pression, comprenant un fluide et relié à un boîtier (1) qui débouche dans un canal (4) avec une ouverture distale (11) à travers laquelle la matière filiforme se trouvant dans le boîtier (1) et s'étendant à travers le canal (4) est transportée hors du dispositif à l'aide de l'agent générant de la pression et du fluide,
**caractérisé en ce que**
l'ouverture distale (11) comprend un rétrécissement à travers lequel peut passer la matière filiforme et qui enveloppe la matière filiforme de manière à ce que le fluide reste dans le dispositif, la matière filiforme étant déplacée à travers le rétrécissement par le fait que l'agent générant une pression génère une pression statique à l'aide du fluide dans l'espace intérieur du dispositif.

2. Dispositif selon la revendication 1,
**caractérisé en ce que**
l'extrémité distale du canal (4) est dotée d'une garniture empêchant la fuite du fluide hors du canal.

3. Dispositif selon la revendication 2,
**caractérisé en ce que**
la garniture est conçue sous la forme d'un joint à lèvre (35).

4. Dispositif selon la revendication 2,
**caractérisé en ce que**
la garniture se compose d'un insert (36).

5. Dispositif selon la revendication 4,
**caractérisé en ce que**
l'insert (36) est appliqué par ajustage serré, soudure, fermeture géométrique ou par des arêtes recourbées.

6. Dispositif selon la revendication 2, 4 ou 5,
**caractérisé en ce que**
la garniture se compose d'une étampe universelle (38).

7. Dispositif selon la revendication 2,
**caractérisé en ce que**
la garniture (42) se compose d'une diminution du canal (4) dans la zone de l'extrémité distale (11) du canal (4).

8. Dispositif selon la revendication 7,
**caractérisé en ce qu'**
une arête (43) du canal est déformée en direction de l'espace intérieur du canal.

9. Dispositif selon la revendication 2,
**caractérisé en ce que**
la garniture (45) est formée par un rétrécissement du canal (4) à proximité de l'ouverture distale (11) du canal.

10. Dispositif selon la revendication 2, 4 ou 5,
**caractérisé en ce que**
la garniture est conçue sous la forme d'un insert (48) conique se rétrécissant vers l'extérieur.

11. Dispositif selon la revendication 2 ou 4,
**caractérisé en ce que**
la garniture est conçue en tant que joint torique d'étanchéité.

12. Dispositif selon l'une quelconque des revendications 1-11,
**caractérisé en ce que**
l'ouverture distale (11) du canal (4) est biaisée vers une pointe (46).

13. Dispositif selon l'une quelconque des revendications 1-12,
**caractérisé en ce qu'**
un agent de séparation du fil se trouve à proximité de l'ouverture (11) du canal (4).

14. Dispositif selon la revendication 13,
**caractérisé en ce que**
l'agent de séparation du fil (9) comprend un cylindre (16) avec l'un des alésages (17, 52) guidant le fil, la rotation de ce cylindre cisaillant le fil (9) au niveau du boîtier du cylindre.

15. Dispositif selon la revendication 13,
**caractérisé en ce que**
la séparation du fil (9) a lieu avec un boulon (18) qui est introduit dans le canal (4) par le côté.

16. Dispositif selon l'une quelconque des revendications 1-12,
**caractérisé en ce qu'**
un agent de séparation du fil (9) se trouve dans la zone de l'ouverture distale (11) du canal (4).

17. Dispositif selon la revendication 16,
**caractérisé en ce que**
l'agent de séparation du fil (9) comprend un insert (20) cylindrique avec un alésage (21) qui peut pivoter à l'aide d'un fil transmetteur (23).

18. Dispositif selon les revendications 16,
**caractérisé en ce que**
l'agent de séparation du fil (9) comprend une plaquette (27) avec un trou (54), laquelle peut être déplacée à l'aide d'un fil transmetteur (23).

19. Dispositif selon la revendication 16, 17 ou 18,
**caractérisé en ce qu'**
un canal (24) à double lumen est utilisé, le fil transmetteur (23) traversant l'un des deux lumens (26) et le fil (9) traversant l'autre lumen (25).

20. Dispositif selon la revendication 16,
**caractérisé en ce que**
l'agent de séparation du fil (9) est un fil métallique (29) et la séparation du fil (9) a lieu en avançant le fil métallique (29) et à l'aide de l'extrémité distale (59) du fil métallique (29).

21. Dispositif selon l'une quelconque des revendications 16 à 20,
**caractérisé en ce que**
le canal (4) est refermé en même temps que la séparation du fil (9).

22. Dispositif selon l'une quelconque des revendications 16 à 20,
**caractérisé en ce qu'**
une ou plusieurs arêtes coupantes sont appliquées avec des moyens propres, de manière à ce que le fil soit coupé lorsqu'il est retiré de manière proximale à travers le canal (4).

23. Dispositif selon la revendication 15 ou 21,
**caractérisé en ce que**
l'agent de séparation du fil (9) comprend une plaquette (55) courbée qui comporte, sur son côté tourné vers l'ouverture, deux arêtes vives en contact avec le fil.

24. Dispositif selon l'une quelconque des revendications 1-23,
**caractérisé en ce qu'**
un agent de fermeture se trouve sur l'extrémité distale du canal (4), lequel agent de fermeture ferme le canal (4) en retenant le fluide s'y trouvant après la sortie de l'extrémité proximale du fil (49) hors du canal (4).

25. Dispositif selon la revendication 24,
**caractérisé en ce que**
le rétrécissement distal du canal (4) est fermé par une sphère (31).

26. Dispositif selon la revendication 24,
**caractérisé en ce que**
l'agent de fermeture comporte une plaquette (33) pouvant pivoter, laquelle plaquette se ferme après la sortie de l'extrémité proximale de fil (49) dans la direction de l'ouverture distale (11) du canal (4) et le ferme.

27. Dispositif selon l'une quelconque des revendications 1 à 26,
**caractérisé en ce qu'**
une soupape d'arrêt (34) est commutée après l'agent générateur de pression qui se ferme après la sortie de l'extrémité proximale de fil (49) hors du canal (4) en raison de l'augmentation du courant de fluide et donc empêche une injection du fluide dans le corps.
